# EUROPEAN PATENT APPLICATION

(11) **EP 3 892 266 A1**
(43) Date of publication of application: **13.10.2021**
(21) Application number: 21176202.6
(22) Date of filing: 13.03.2014
(51) Int. Cl.: A61K 31/121, A61K 31/198, A61K 31/277, A61P 25/16, A61P 43/00

(54) **METHOD FOR TREATMENT OF PARKINSON'S DISEASE**

(30) Priority: 13.03.2013 US 201361779357 P
(62) Divisional of application: 14718177.0
(71) Applicant: Neuroderm Ltd., 7403648 Ness Ziona (IL)
(72) Inventor: Yacoby-Zeevi, Oron, 6094600 Bitsaron (IL)
(74) Representative: Hoffmann Eitle

(57) **Abstract**

The present invention provides a method for treatment of neurological or movement disorders such as Parkinson's disease by parenteral administration of levodopa and carbidopa, concomitantly with oral administration of a COMT inhibitor such as entacapone and tolcapone.

## Description

### TECHNICAL FIELD

The present invention provides a method for treatment of neurological or movement disorders such as Parkinson's disease by parenteral administration of levodopa and carbidopa, concomitantly with oral administration of a COMT inhibitor such as entacapone and tolcapone.

### BACKGROUND

Parkinson's disease is a degenerative condition characterized by reduced concentration of the neurotransmitter dopamine in the brain. Levodopa (L-dopa or L-3,4-dihydroxyphenylalanine) is an immediate metabolic precursor of dopamine that, unlike dopamine, is able to cross the blood-brain barrier, and is most commonly used for restoring the dopamine concentration in the brain. For the past 40 years, levodopa has been remained the most effective therapy for treatment of Parkinson's disease.

However, levodopa has a short half-life in plasma that, even under best common current standard of care, results in pulsatile dopaminergic stimulation. Long-term therapy is therefore complicated by motor fluctuations and dyskinesia that can represent a source of significant disability for some patients. A therapeutic strategy that could ultimately deliver levodopa/dopamine to the brain in a more continuous and physiologic manner would provide the benefits of standard levodopa with reduced motor complications and is much needed by patients suffering from Parkinson's disease and other neurological or movement disorders (Olanow, Mov. Dis., 2008, 23(Suppl. 3), S613-S622). Sustained-release oral levodopa formulations have been developed but, at best, such preparations have been found to be no more efficacious than standard tablets. Continuous administration of levodopa by intraduodenal administration or infusion has also been attempted by using ambulatory pumps or patches. Such treatments, especially intraduodenal, are extremely invasive and inconvenient.

The metabolic transformation of levodopa to dopamine is catalyzed by the aromatic L-amino acid decarboxylase enzyme, a ubiquitous enzyme with particularly high concentrations in the intestinal mucosa, liver, brain and brain capillaries. Due to the extracerebral metabolism of levodopa, it is necessary to administer large doses of levodopa leading to high extracerebral concentrations of dopamine that cause unwanted side effects in some of the patients. Therefore, levodopa is usually administered concurrently with oral administration of a dopa decarboxylase inhibitor, such as carbidopa or benserazide, which reduces by 60-80% the levodopa dose required for a clinical response, and thus prevents certain of its side effects by inhibiting the conversion of levodopa to dopamine outside of the brain.

Additional extracerebral metabolic pathways of levodopa are through monoamine oxidase (MAO), or catechol-O-methyl transferase (COMT) which converts levodopa to 3-methoxy-4-hydroxy-L-phenylalanine (3-O-methyldopa, 3-OMD), a metabolite that competes with levodopa in crossing the blood-brain barrier. Therefore, MAO inhibition by, e.g., moclobemide, rasagiline, selegiline or safinamide, or COMT inhibition by, e.g., entacapone or tolcapone, improves the efficacy of levodopa. Entacapone exhibits short biphasic elimination, with a β-phase half-life of about 0.4 to about 0.7 hours and a γ-phase half-life of about 2.4 hours and is therefore typically administered six to eight times per day. Tolcapone binds to the catalytic center of COMT in both peripheral and central nervous systems with greater affinity than any of the three catecholamine, including levodopa, and consequently prevents the 3-O-methylation of levodopa by COMT. Tolcapone thus improves the bioavailability and reduces the clearance of levodopa and subsequently dopamine from the central nervous system.

The efficacy of levodopa's metabolic pathways inhibitors, when concomitantly administered with levodopa, in increasing the level of the latter in the blood is well documented, and various formulations of levodopa together with such inhibitors have been disclosed. For example, currently available oral drugs include SINEMET® and sustained-release SINEMET® CR tablets that include levodopa and carbidopa, as well as MADOPAR® tablets containing levodopa and benserazide. Additional formulations of levodopa and carbidopa for subcutaneous, preferably continuous, administration are disclosed in International Publication Nos. WO 2010/134074 and WO 2012/066538, the entire contents of each being herewith incorporated by reference in its entirety as if fully disclosed herein.

An additional oral drug is STALEVO® tablets containing levodopa, carbidopa and entacapone, and formulations of these ingredients, e.g., for subcutaneous administration, are disclosed in the aforesaid WO 2012/066538, which further discloses oral administration of SINEMET® and concomitant continuous subcutaneous administration of either entacapone or entacapone and carbidopa.

Nyholm et al. (European Journal of Neurology, 2012, 19, 820-826) shows that oral administration of 200 mg entacapone and concomitant intraintestinal infusion of levodopa and carbidopa at a dosage equal to 80% of the optimized dose of a patient, allows maintaining a blood level of levodopa that is substantially the same as that achieved by administration of the optimized dose of levodopa and carbidopa, without entacapone. In other words, Nyholm *et al.* shows that oral administration of entacapone allows reducing the initial dose of levodopa and carbidopa administered by 20% and still maintain the desired blood level of levodopa.

Yet, the various formulations disclosed do not provide the optimal treatment of Parkinson's disease due to still relatively high level of side effects and levodopa blood level fluctuations. There is thus an ongoing and urgent need for methods for treatment of movement disorders such as Parkinson's, that will provide patients with a constant blood levodopa level that will lead to constant dopaminergic stimulation in the brain, and at the same time limit the side effects caused by high peripheral levodopa concentration resulting from high dose levodopa administration.

### SUMMARY OF INVENTION

In one aspect, the present invention provides a method for treatment of Parkinson's disease in an individual in need thereof comprising parenterally administering a pharmaceutical composition comprising carbidopa and levopoda, or pharmaceutically acceptable salts thereof, to said individual; and orally administering a catechol-O-methyl transferase (COMT) inhibitor such as entacapone or tolcapone.

In another aspect, the present invention relates to carbidopa or a pharmaceutically acceptable salt thereof, levodopa or a pharmaceutically acceptable salt thereof, and a COMT inhibitor for use as a combination in treatment of Parkinson's disease, wherein the carbidopa and the levodopa (or salts thereof) are formulated as a sole parenteral composition, and said COMT inhibitor is formulated as an oral composition.

### BRIEF DESCRIPTION OF THE FIGURES

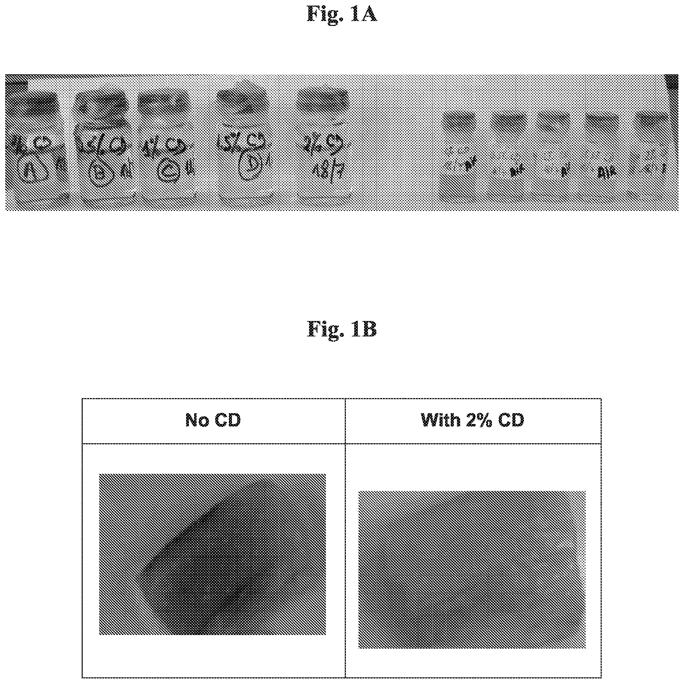

**Figs. 1A-1B** show the effect of carbidopa on the stability of levodopa *in vitro* and *ex vivo:* **1A.** 6% by weight levodopa and arginine solution with various concentrations (2, 1.5, 1, 0.5%) of carbidopa or no carbidopa were tested for physical stability *in vitro.* The results show that carbidopa prevented dark yellow color formation in the presence of air, in a dose related manner (small vials at the right hand side), and in the absence of air (with N₂ in the head space) 0.5% carbidopa was sufficient to inhibit this color formation (large vials in the left hand side of the figure). **1B.** 7% by weight levodopa and arginine solution, with or without 2% carbidopa by weight, continuously administered in to the subcutaneous tissue of a 5×5cm fresh, full-thickness pig skin. The right hand side depicts the inhibition of oxidation with the use of a levodopa formulation that includes carbidopa.

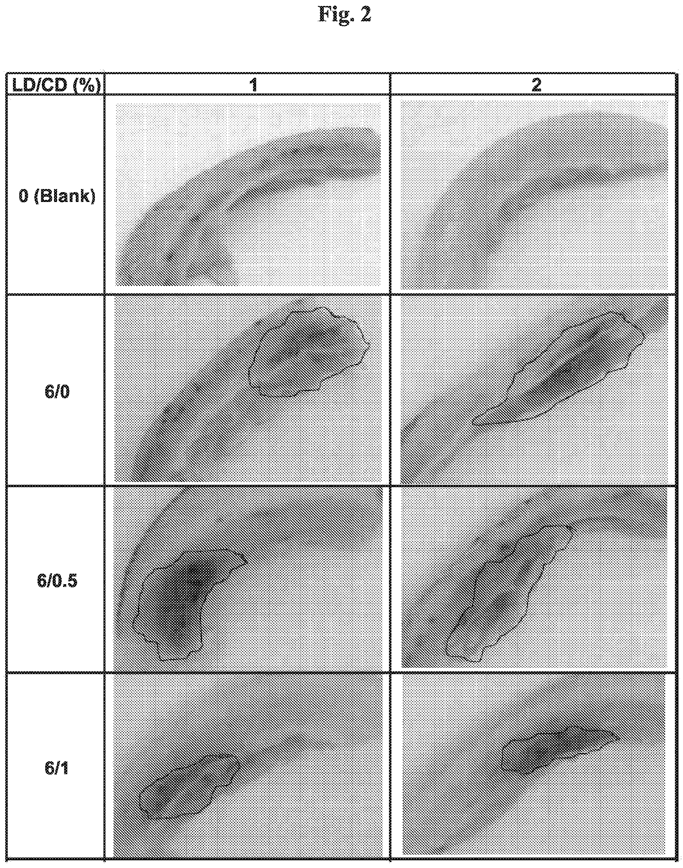

**Fig. 2** shows that the presence of 1% carbidopa in a levodopa solution reduces the severity and extent of local levodopa dependent subcutaneous toxicity in the pig.

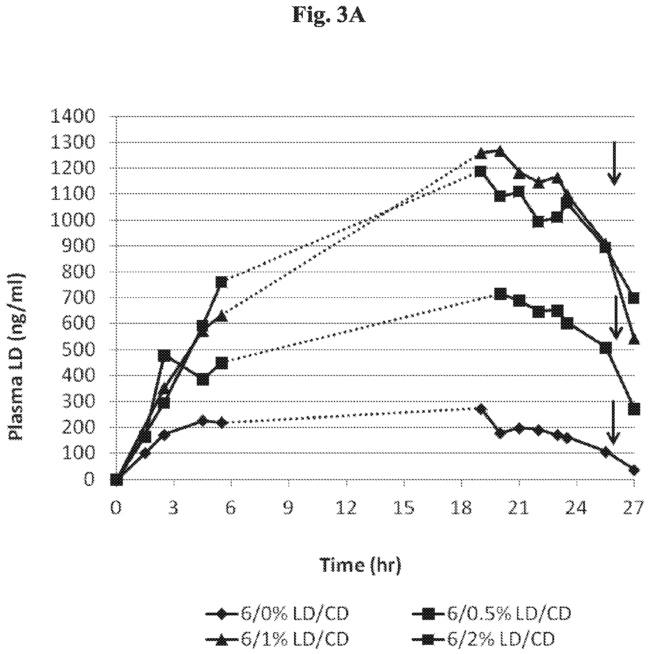

**Figs. 3A-3C** show the effect of carbidopa on the pharmacokinetics of levodopa in the pig. **3A:** the plasma concentration of levodopa following continuous subcutaneous administration of 6% levodopa with various amounts of carbidopa. **3B:** The correlation between plasma steady state concentrations of levodopa, obtained following continuous subcutaneous administration of levodopa/carbidopa formulations and the formulation concentration of carbidopa. **3C:** The correlation between plasma steady state concentration of carbidopa following continuous subcutaneous administration of levodopa/carbidopa formulations and the formulation concentration of carbidopa.

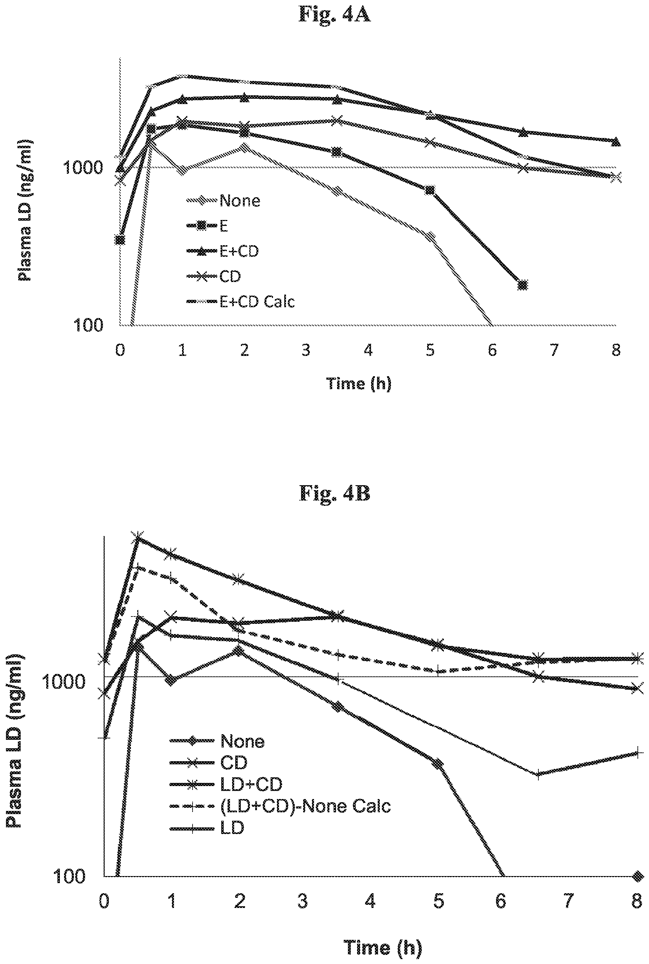

**Figs. 4A-4B** show the effect of continuous subcutaneous (SC) entacapone (E) and/or carbidopa (CD) (40 mg/24 h) administration on the plasma concentrations of levodopa (LD, ng/ml) following oral administration of Sinemet (100/25 levodopa/ carbidopa) in pigs **(4A);** and the effect of continuous SC CD (40 mg/24 h) and/or LD (140 mg/24 h) administration on the plasma concentrations of LD following oral administration of Sinemet in pigs **(4B).**

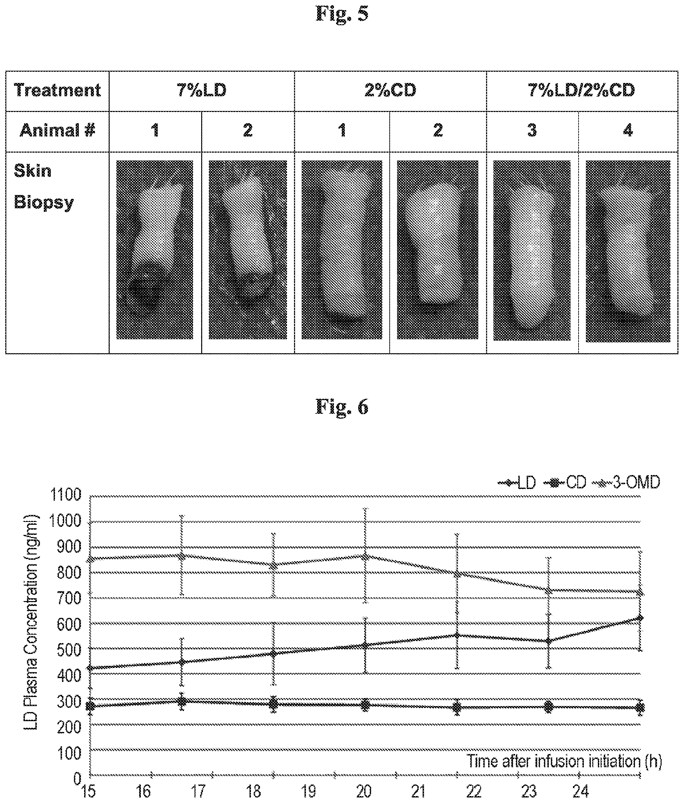

**Fig. 5** shows the effect of carbidopa on the local subcutaneous toxicity of levodopa following 24 h continuous subcutaneous administration, at 0.16 ml/h, in pigs.
**Fig. 6** show the effect of oral administration of entacapone (200 mg every 2 h) on the plasma concentrations of levodopa (LD), carbidopa (CD), and 3-O-methyldopa (3-OMD) during continuous SC administration of levodopa (360 mg/24 h) and carbidopa (90 mg/24 h) in human volunteers.

### DETAILED DESCRIPTION OF THE INVENTION

As stated above, the concomitant administration of levodopa and an active agent capable of inhibiting a metabolic pathway of levodopa is well known, and one of the commercially available drugs for treatment of Parkinson's disease is STALEVO® that, in fact, comprises a combination of levodopa, carbidopa and entacapone. Nevertheless, despite of the advantages associated with a joint oral administration of the three ingredient, this specific route of administration has several drawbacks because oral administration results in fluctuations of blood levodopa level; and entacapone, when present in one solution with carbidopa, e.g., after dissolution of a tablet in the gastrointestinal tract), reduces carbidopa's bioavailability. This is clearly indicated in Ahtila et al. (Clin Neuropharmacol., 1995, 18(1), 46-57), demonstrating that high doses of entacapone (400 mg and 800 mg), concomitantly orally administered with levodopa and carbidopa, decrease the total amount of carbidopa in blood but do not affect the time to maximal concentration (Tₘₐₓ) of carbidopa. Ahtila *et al.* also show that the highest increase in the total amount of levodopa caused by the addition of entacapone to oral administration of levodopa and carbidopa was about 33% (after 400 mg dose of entacapone).

The aforesaid suggests that it might be beneficial to administer carbidopa separately from entacapone, i.e., using either different administration routes or by administering them in two different locations of the patient's body. Indeed, as already shown by the present inventors and shown in WO 2012/066538, oral administration of Sinemet® (levodopa/carbidopa, 100/25 mg) and concomitant subcutaneous administration of either entacapone or a combination of entacapone and carbidopa (formulated as different compositions) resulted in increased blood levels of levodopa. As specifically shown, entacapone and carbidopa, when continuously subcutaneously co-administered with Sinemet®, act in synergy on the plasma pharmacokinetics of levodopa.

In another approach disclosed in the aforesaid Nyholm *et al.,* entacapone is orally administered while a combination of levodopa and carbidopa is administered concomitantly by intraintestinal infusion, and as shown, oral administration of entacapone allows reducing the initial dose of levodopa and carbidopa administered by 20% while maintaining the desired blood level of levodopa. According to Nyholm *et al.,* the administration route disclosed may allow an increase of up to 33% in the level of levodopa.

It has now been found, in accordance with the present invention, that continuous subcutaneous administration of a composition comprising both levodopa and carbidopa, and concomitant oral administration of a catechol-O-methyl transferase inhibitor, in particular, entacapone, to Parkinson's patients unexpectedly increases the blood level of levodopa by more than 40%, more particularly from about 420 ng/ml to about 620 ng/ml, while maintaining a constant blood level of levodopa with no substantial fluctuations, and therefore allows significantly reducing the initial dose of levodopa administered to Parkinson's patients and subsequently reducing the side effects of the drug. Moreover, the experimental data provided herein show that the subcutaneous administration of a sole composition comprising levodopa and carbidopa have additional benefits compared with separate administrations thereof due to reduced local side effects caused by levodopa when administered together with carbidopa.

These findings show that oral administration of a COMT inhibitor concomitantly with a parenteral, e.g., subcutaneous, administration, in particular continuous parenteral administration, of levodopa and a dopa decarboxylase inhibitor such as carbidopa leads to levodopa blood level that is remarkably higher than those shown in any one of the prior art utilizing a different administration regimen, suggesting that the administration regimen demonstrated herein allows to utilize to the fullest the inhibitory potentials of both inhibitors.

The present invention thus provides a strategy for treatment of a neurological or movement disorder such as Parkinson's disease, comprising parenteral (e.g., continuous) administration of a combination of levodopa and a dopa decarboxylase inhibitor, or pharmaceutically acceptable salts of the aforesaid, concomitantly with oral administration of a COMT inhibitor. Such a strategy allows providing high plasma levodopa level thereby substantially continuous stimulation of the dopaminergic system in the brain, and may allow decreasing the COMT inhibitor oral dosing regimen from 5-8 times/day to 2-3 times/day, and/or reduce daily dose of levodopa and/or COMT inhibitor.

A neurological disorder is a disorder of the body's nervous system, and the term "movement disorder" as used herein refers to a nervous system condition that causes abnormal voluntary or involuntary movements, or slow, reduced movements.

The neurological or movement disorder treatable according to the treatment strategy disclosed herein includes, without being limited to, Parkinson's disease, secondary parkinsonism, restless leg syndrome, Huntington's disease, Shy-Drager syndrome, and dystonia, as well as various conditions resulting from brain injury such as, without limiting, carbon monoxide or manganese intoxication. In one embodiment, the neurological disease to be treated according to this strategy is Parkinson's disease.

The term "dopa decarboxylase inhibitor" as used herein refers to an agent capable of inhibiting the peripheral metabolism of levodopa to dopamine by aromatic L-amino acid decarboxylase such as carbidopa and benserazide. In a particular embodiment, the dopa decarboxylase inhibitor used according to the treatment strategy disclosed herein is carbidopa or a pharmaceutically acceptable salt thereof.

The term "catechol-O-methyl transferase (COMT) inhibitor" as used herein refers to an agent capable of inhibiting the degradation of levodopa to 3-O-methyldopa (3-OMD) by catechol-O-methyl transferase and thus prolonging the half-life of levodopa in the circulation. Examples of COMT inhibitors include, without being limited to, entacapone, that is only peripherally active, as well as tolcapone and nitecapone that are active in both the periphery and central nervous system. Preferred COMT inhibitors to be administered according to the treatment strategy disclosed herein are entacapone and tolcapone. The dose of COMT inhibitor administered is determined so as to allow obtaining substantially constant inhibition of COMT activity upon administration, thereby increasing the half-life of administered levodopa and substantially reducing the pulsatility of levodopa plasma levels to avoid low trough levels of plasma levodopa.

According to the treatment strategy disclosed herein, the combination of levodopa and dopa decarboxylase inhibitor, e.g., carbidopa, parenterally administered to the treated individual, may be formulated as two separate pharmaceutical compositions (herein ***"the parenteral compositions"***) wherein one of said compositions comprises levodopa or a pharmaceutically acceptable salt thereof, and another of said compositions comprises said dopa decarboxylase inhibitor or a pharmaceutically acceptable salt thereof; or as a sole pharmaceutical composition comprising both active components (herein ***"the parenteral composition"***). In a particular embodiment, the combination of levodopa and dopa decarboxylase inhibitor is formulated as a sole pharmaceutical composition that is parenterally administered to the treated individual; and the COMT inhibitor, e.g., entacapone or tolcapone, is formulated as a pharmaceutical composition orally administered to said individual (herein ***"the oral composition"***).

The term "pharmaceutical composition" as used herein refers to a composition comprising at least one active component as disclosed herein formulated together with one or more pharmaceutically acceptable carriers or pharmaceutically acceptable excipients. The terms "pharmaceutically acceptable carrier" and "pharmaceutically acceptable excipient" as used herein refer to any and all solvents, dispersion media, preservatives, antioxidants, coatings, isotonic and absorption delaying agents, and the like, that are compatible with pharmaceutical administration. The use of such media and agents for pharmaceutically active substances is well known in the art. The compositions may also contain other active compounds providing supplemental, additional, or enhanced therapeutic functions. "Pharmaceutically or pharmacologically acceptable" include molecular entities and compositions that do not produce an adverse, allergic or other untoward reaction when administered to an animal, or a human, as appropriate. For human administration, preparations should meet sterility, pyrogenicity, safety and purity standards as required by, e.g., the U.S. FDA Office of Biologics standards.

In one aspect, the present invention thus provides a method for treatment of a neurological or movement disorder such as Parkinson's disease in an individual in need thereof comprising parenterally administering a pharmaceutical composition comprising carbidopa and levopoda, or pharmaceutically acceptable salts thereof, to said individual; and orally administering to said individual a COMT inhibitor. In an alternative method, carbidopa and levodopa, or pharmaceutically acceptable salts thereof, are parenterally administered from two different pharmaceutical compositions, wherein one of those compositions comprises carbidopa or a pharmaceutically acceptable salt thereof, and another one of said compositions comprises levodopa or a pharmaceutically acceptable salt thereof.

The parenteral composition may be administered by any parenteral administration route, e.g., subcutaneously, transdermally, intradermally, intravenously, intramuscularly, intratracheally, intranasally, intrathecally, intragastrically or intraduodenally, and in one or more sites on the individual's body. In particular embodiments, the parenteral composition is substantially continuously administered, e.g., substantially continuously subcutaneously, transdermally, intradermally, intragastrically or intraduodenally administered to said individual. Continuous administration of the parenteral composition may be accomplished using, e.g., an infusion pump, or a dermal patch, namely, a device suitable for transdermal or subcutaneous administration of the parenteral composition, i.e., capable of delivering said composition through the skin or mucous membrane into the bloodstream of a patient. Dermal patches suitable for use according to the method of the invention may have one or more compartments containing the parenteral composition or compositions to be administered, and are described, e.g., in the aforesaid WO 2012/066538.

The weight ratio between the levodopa and dopa decarboxylase inhibitor, e.g., carbidopa, administered according to the method of the present invention may be any weight ratio that, together with the COMT inhibitor concomitantly administered, would be sufficient to maintain therapeutic plasma levels of levodopa, no matter whether said levodopa and dopa decarboxylase inhibitor are formulated as a sole parenteral composition or as two separate parenteral compositions. In certain embodiments, the levodopa: carbidopa weight ratio is in a range of about 20:1 to about 1:1.

In certain embodiments, the parenteral composition further comprises arginine. According to particular such embodiments, the parenteral composition comprises carbidopa and levodopa in an overall molar ratio to arginine of about 1:2 to about 1:3.5. As previously shown by the present inventors and disclosed in WO 2010/134074 and WO 2012/066538, such compositions are significantly more stable than compositions where no arginine is used, or compositions where basic amino acid other than arginine, e.g., lysine or histidine, are used.

In some particular such embodiments, the parenteral composition according to the method of the invention comprises (i) arginine, about 0.1% to about 2% by weight carbidopa, and about 4% to about 8% by weight levodopa; or (ii) arginine, about 0.6% to about 1.5% by weight carbidopa, and about 6% by weight levodopa. Such compositions may be administered at a rate of about 0.1 to about 1000 µl/hour/site; or at a volume of about 2 to about 10 ml/24hour/site, e.g., about 4 to about 6 ml/24hour/site; or at a dose of about 80 to about 800 mg levodopa/day and about 20 to about 200 mg carbidopa/day; or at a rate of about 240 to about 360 mg levodopa and about 60 to about 90 mg carbidopa/day/site. More specific such parenteral compositions are administered subcutaneously, transdermally, intradermally, intravenously, intramuscularly, intratracheally, intranasally or intrathecally.

In other particular such embodiments, the parenteral composition comprises (i) arginine, about 1% to about 4% by weight carbidopa, and about 6% to about 16% by weight levodopa; or (ii) arginine, about 1.5% to about 2.5% by weight carbidopa, and about 12% by weight levodopa. Such compositions may be administered at a rate of about 0.2 to about 2000 µl/hour/site; or at a volume of about 10 to about 24 ml/24hour/site, e.g., about 12 to about 16 ml/24hour/site; or at a dose of about 600 to about 4000 mg levodopa/day and about 60 to about 500 mg carbidopa/day; or at a rate of about 800 to about 1600 mg levodopa and about 200 to about 400 mg carbidopa/day/site. More specific such parenteral compositions are administered intragastrically or intraduodenally.

In certain particular embodiments, the parenteral composition according to the method of the invention comprises arginine, levodopa and carbidopa, wherein the parenteral composition has levodopa:arginine molar ratio selected from about 1:1.5 to about 1:2.5, respectively, and a pH of about 8.5 to about 10, i.e., about 8.5, 8.6, 8.7, 8.8, 8.9, 9.0, 9.1, 9.2, 9.3, 9.4, 9.5, 9.6, 9.7, 9.8, 9.9 or 10, at 25°C. Such compositions may comprise, e.g., about 1% to about 20% or more, by weight, carbidopa, preferably at least about 1%, 2%, 4% or 6% by weight carbidopa; or at least about 1%, preferably about 2%, 3%, 4%, 5% or 6%, by weight levodopa. More particular such compositions may further comprise a pharmaceutically acceptable excipient such as N-methylpyrrolidone, polyvinylpyrrolidone, propylene glycol, or a combination thereof, and may still further comprise water. In specific embodiments, such parenteral compositions have a pH of about 8.5 to about 9.8 at 25°C.

In certain particular embodiments, the parenteral composition according to the method of the invention comprises arginine, carbidopa, at least about 4% by weight levodopa, and optionally meglumine, wherein the parenteral composition has a pH of about 9.1 to about 9.8, i.e., about 9.1, 9.2, 9.3, 9.4, 9.5, 9.6, 9.7 or 9.8, at 25°C. Such compositions may have a molar ratio of active components, i.e., carbidopa and levodopa, to arginine of about 1:1.8 to about 1:3.5, or about 1:2.2 to about 1:2.5. More particular such compositions comprise about 4% to about 12%, or about 5% to about 30%, by weight levodopa, and/or about 1% to about 6%, or about 1% to about 2%, by weight carbidopa.

In some embodiments, the parenteral composition comprises arginine, carbidopa, and at least about 4% by weight levodopa, as defined hereinabove, and further comprises meglumine. Certain particular such compositions are those wherein the molar ratio of active components to the arginine is about 1:1.1 to about 1:1.9, and/or the molar ratio of active components to the meglumine is about 1:0.3 to about 1:1.2, about 1:0.3 to about 1:1.5, about 1:0.4, or about 1:1.1. Other particular such compositions are those comprising about 2.0% to about 11% by weight meglumine, and/or about 10% to about 35% by weight arginine.

In more particular embodiments, the parenteral composition comprises arginine, carbidopa, at least about 4% by weight levodopa, and optionally meglumine, as defined in any one of the embodiments above, and further comprises at least one agent capable of inhibiting the formation of oxidation products, e.g., ascorbic acid or a pharmaceutically acceptable salt thereof such as sodium ascorbate, calcium ascorbate, potassium ascorbate, ascorbyl palmitate, or ascorbyl stearate, L-cysteine, N-acetylcysteine (NAC), gluthatione (GSH), Na₂-EDTA, Na₂-EDTA-Ca, or a combination thereof.

According to the present invention, parenteral compositions comprising arginine, carbidopa, at least about 4% by weight levodopa, and optionally meglumine, as defined in any one of the embodiments above, may further comprise sodium bisulfite.

In certain specific embodiments, the parenteral composition according to the method of the present invention comprises about 6% by weight levodopa, about 0.6% to about 1.4% by weight carbidopa, about 15% to about 16% by weight arginine, and about 0.5% by weight ascorbic acid; may further comprise about 0.4% by weight L-cysteine and/or about 0.5% by weight NAC; and has a pH of about 9.4 to about 9.6.

In other specific embodiments, the parenteral composition according to the method of the present invention comprises about 12% to about 15% by weight levodopa, about 1.2% to about 4% by weight carbidopa, about 32% to about 42% by weight of either arginine or meglumine; and about 1.0% to about 1.3% by weight sodium ascorbate; may further comprise about 0.1% to about 0.5% by weight L-cysteine and/or NAC and/or cysteine-HCl; and has a pH of about 9.6 to about 9.8.

According to the present invention, a parenteral composition having any one of the various compositions defined above is administered concomitantly with an oral composition comprising a COMT inhibitor. The COMT inhibitor may be administered at a dosage of, e.g., about 10 to about 1600 mg per day, about 50 to about 400 mg per day, about 100 to about 600 mg per day, about 400 to about 1200 mg per day, about 1000 to about 1400 mg per day, or about 1200 mg to about 1600 mg per day. The oral composition may be administered 1, 2, 3, 4 or 5 times a day. In particular embodiments, the COMT inhibitor is administered at a lower frequency than presently recommended, e.g., two to three times per day, or in a lower daily dosage amount.

In certain embodiments, the oral composition comprises entacapone, and it is administered concomitantly with the parenteral composition at a dose of about 200 mg to about 600 mg, e.g., about 200, 250, 300, 350, 400, 450, 500, 550 or 600 mg, twice or three times a day. In more particular such embodiments, the oral composition is administered at a dose of about 350 to about 450 mg, e.g., about 400 mg, twice a day.

In other embodiments, the oral composition comprises tolcapone, and it is administered concomitantly with the parenteral composition at a dose of about 50 mg to about 200 mg, e.g., about 50, 75, 100, 125, 150, 175 or 200 mg, once, twice or three times a day. In more particular such embodiments, the oral composition is administered at a dose of about 75 to about 125 mg, e.g., about 100 mg, twice a day.

Oral compositions of the COMT inhibitor may be in any suitable form. For example, the oral composition may be formulated as a pill, hard or soft capsule, tablet, troches, lozenges, aqueous or oily suspensions, dispersible powders or granules, emulsions, syrups or elixirs. The oral formulation may comprise, e.g., about 200 mg to about 600 mg, about 50 mg to about 200 mg, or 100 mg, of the COMT inhibitor per dose. In some cases, the oral formulation may be a controlled release formulation, i.e., formulated for controlled (sustained, extended, or prolonged) or delayed release of the COMT inhibitor.

The various pharmaceutical compositions utilized according to the method of the invention may be prepared by conventional techniques, e.g., as described in Remington: The Science and Practice of Pharmacy, 19^{th} Ed., 1995. The compositions can be prepared, e.g., by uniformly and intimately bringing the active component/components into association with a liquid carrier, a finely divided solid carrier, or both, and then, if necessary, shaping the product into the desired formulation. The compositions may be in liquid, solid or semisolid form and may further include pharmaceutically acceptable fillers, carriers, diluents or adjuvants, and other inert ingredients and excipients. In one embodiment, the pharmaceutical composition of the present invention is formulated as nanoparticles.

The parenteral compositions utilized may be prepared by mixing levodopa and a dopa decarboxylase inhibitor, e.g., carbidopa, optionally together with arginine and/or meglumine and/or one or more antioxidants, in amounts as disclosed above, to form a powder mixture. Water may be added to the mixture to form a suspension. The suspension may be heated, e.g., to about 60-90°C, more particularly to about 72±5°C, e.g., by adding pre-heated water and/or by placing the mixture in a hot (e.g., 72±5°C) water bath for a sufficient time period, e.g., for about 3 minutes, about 5 minutes, about 10 minutes or more, to form a solution, with optional stirring, and cooling the solution to form the composition. N₂ may be provided the head space of the container. For example, the mixture can be removed from the hot water bath and cooled to room temperature (RT), and an antioxidant may then be added under N₂ atmosphere and subsequent stirring. A preparation such as that above, e.g., where levodopa, carbidopa, and arginine are mixed together as powders first, and a suspension with water is then formed and heated, may result in a more stable solution as compared to a preparation that includes a step wise preparation of individual water suspensions of ingredients and later combination.

The parenteral compositions may be sterilized, e.g., using 0.2 µM filters such as filters with nylon or polyvinylidene difluoride (PVDF) membranes. In some embodiments, the compositions have fewer undesirable by-products, e.g., toxic by-products, or contaminants, e.g., hydrazine, when carbidopa and levodopa are present at the same time, and when prepared using certain antioxidants, e.g., ascorbic acid or salts thereof, rather than others, e.g., sodium bisulfite. In other embodiments, the compositions have fewer undesirable by-products when pre-heated water is added as disclosed above, as compared to formulations prepared without the addition of pre-heated water. In further embodiments, the levodopa and/or carbidopa may not dissolve unless the preparation procedure described above is used. Such preparations as described above may provide more stable formulations as compared to formulations prepared without adding hot water or heating.

Oral compositions of the COMT inhibitor may be prepared according to any method known in the art for the manufacture of pharmaceutical compositions and may further comprise one or more ingredients selected from sweetening agents, flavoring agents, coloring agents and preserving agents in order to provide pharmaceutically elegant and palatable preparations. Tablets contain the active component, i.e., the COMT inhibitor, in admixture with non-toxic pharmaceutically acceptable excipients, which are suitable for the manufacture of tablets. These excipients may be, e.g., inert diluents such as calcium carbonate, sodium carbonate, lactose, calcium phosphate, or sodium phosphate; granulating and disintegrating agents, e.g., corn starch or alginic acid; binding agents, e.g., starch, gelatin or acacia; and lubricating agents, e.g., magnesium stearate, stearic acid, or talc. The tablets may be either uncoated or coated utilizing known techniques to delay disintegration and absorption in the gastrointestinal tract and thereby provide a sustained action over a longer period. For example, a time delay material such as glyceryl monostearate or glyceryl distearate may be employed. They may also be coated using the techniques described in the US Patent Nos. 4,256,108, 4,166,452 and 4,265,874 to form osmotic therapeutic tablets for control release. The oral compositions may also be in the form of oil-in-water emulsion.

The oral compositions may be formulated for either immediate or controlled release of the COMT inhibitor. Such controlled release compositions may be formulated as controlled-release matrix, e.g., as controlled-release matrix tablets in which the release of a soluble active agent is controlled by having the active diffuse through a gel formed after the swelling of a hydrophilic polymer brought into contact with dissolving liquid (*in vitro*) or gastro-intestinal fluid (*in vivo*). Many polymers have been described as capable of forming such gel, e.g., derivatives of cellulose, in particular the cellulose ethers such as hydroxypropyl cellulose, hydroxymethyl cellulose, methylcellulose or methyl hydroxypropyl cellulose, and among the different commercial grades of these ethers are those showing fairly high viscosity. In other embodiments, the compositions comprise the active component formulated for controlled release in microencapsulated dosage form, in which small droplets of the COMT inhibitor are surrounded by a coating or a membrane to form particles in the range of a few micrometers to a few millimeters.

Other contemplated formulations are depot systems, based on biodegradable polymers, wherein as the polymer degrades, the active component is slowly released. The most common class of biodegradable polymers is the hydrolytically labile polyesters prepared from lactic acid, glycolic acid, or combinations of these two molecules. Polymers prepared from these individual monomers include poly (D,L-lactide) (PLA), poly (glycolide) (PGA), and the copolymer poly (D,L-lactide-co-glycolide) (PLG).

The treatment strategy disclosed herein is aimed at increasing the half-life of administered levodopa and substantially reducing the pulsatility of plasma levels thereof, by co-administering levodopa and a dopa decarboxylase inhibitor such as carbidopa, and substantially constantly inhibiting COMT activity, thus providing the individual treated with a constant plasma levodopa level that will lead to constant dopaminergic stimulation in the brain, and at the same time limiting the side effects caused by high plasma levodopa levels resulting from high dose levodopa administration. Contemplated administration of levodopa, a dopa decarboxylase inhibitor, and a COMT inhibitor according to the method of the present invention can typically be carried out over a defined time period, e.g., days, weeks, months and even years, depending on the state of the patient, and as deemed appropriate by the practitioner.

In another aspect, the present invention relates to a dopa decarboxylase inhibitor or a pharmaceutically acceptable salt thereof, levodopa or a pharmaceutically acceptable salt thereof, and a COMT inhibitor, e.g., entacapone or tolcapone, for use as a combination in treatment of a neurological or movement disorder, wherein the dopa decarboxylase inhibitor and the levodopa, or said pharmaceutically acceptable salts thereof, are formulated, either separately or as a combination, as a parenteral composition(s), and said COMT inhibitor is formulated as an oral composition.

In a particular such aspect, the invention relates to carbidopa, levodopa and a COMT inhibitor, more particularly entacapone or tolcapone, for use as a combination in treatment of Parkinson's disease, wherein the carbidopa and the levodopa are formulated as a sole parenteral composition as defined above, and said COMT inhibitor is formulated as an oral composition as defined above.

In certain embodiments, the parenteral composition(s) is formulated as a subcutaneous, transdermal, intradermal, intravenous, intramuscularly, intratracheally, intranasally, intrathecal, intragastrical or intraduodenal composition. Particular such parenteral compositions are suitable for substantially continuous administration.

Also contemplated herein is a kit comprising: (i) a parenteral composition as defined above, i.e., a pharmaceutical composition formulated for parenteral, e.g., transdermal, intradermal or subcutaneous, administration, preferably continuous parenteral administration, said parenteral composition comprising a dopa decarboxylase inhibitor, e.g., carbidopa, and levodopa, or pharmaceutically acceptable salts thereof, e.g., meglumine or arginine salts thereof; (ii) an oral composition as defined above, i.e., a pharmaceutical composition formulated for oral administration, said oral composition comprising a COMT inhibitor, e.g., entacapone or tolcapone; and (iii) instructions for concomitant administration of the pharmaceutical compositions for treatment of a neurological or movement disorder such as Parkinson's disease.

The parenteral composition included in the kit disclosed may be liquid or a lyophilized powder that can be reconstituted into a liquid formulation, or may form part of a dermal patch, and/or may be designed for continuous administration by any suitable parenteral administration route such as transdermally, intravenously, subcutaneously, intradermally, intramuscularly, intragastrically or intraduodenally. The oral composition included in the kit may be formulated for either immediate or controlled release of the COMT inhibitor as described above, and may be in any one of the forms described above.

In certain embodiments, either the parenteral or oral composition included in the kit disclosed, or both, may be provided in a container(s), i.e., a pre-filled cartridge(s), suitable for use by a patient or a physician. For example, provided herein is a kit comprising a prefilled cartridge containing a parenteral liquid composition comprising carbidopa, levodopa and arginine; and an oral composition, e.g., one or more tablets or pills, of a COMT inhibitor, e.g., entacapone or tolcapone; and optionally instructions for use.

The invention now being generally described, will be more readily understood by reference to the following examples which are included merely for purposes of illustration of certain aspects and embodiments of the present invention, and are not intended to limit the invention in any way.

### EXAMPLES

### Example 1. Preparation of solutions/formulation for subcutaneous administration

A. A 2% carbidopa solution/formulation was prepared by adding pre-heated 0.1% Na-bisulfite solution to carbidopa [ASSIA Ltd.]. Arginine (Merck) was added to obtain a final molar ratio of 1:1.2 CD (carbidopa):Arg(arginine). The mixture was stirred at 60°C until complete dissolution was obtained. Heating was stopped and the preparation was allowed to cool down to RT; pH of 8.5. Solution was filtered using a sterile 0.22 µM PVDF membrane.
B. A 10% tolcapone solution/formulation was prepared as follows: a solution containing 10% tolcapone was prepared by adding the respective amount of H₂O to tolcapone (Synfine Research), slowly adding arginine while stirring to obtain a final molar ratio of 1:1. The mixture was stirred until complete dissolution was obtained. After cooling down, the pH of the solution was 7.8.
C. A solution containing 10% entacapone was prepared by adding the respective amount of H₂O to entacapone (Suven Life Sciences), stirring at 30-35°C and slowly adding arginine to obtain a final molar ratio of 1:1. The mixture was stirred until complete dissolution was obtained. After cooling down, the pH of the solution was 6.9. The pH of less concentrated solutions (6%) was 7.8. After preparation, such entacapone solution can be diluted to a 2%, 3% or 4% by weight formulation.
   Entacapone did not dissolve (at concentrations >1%) with other amino acids such as histidine and glutamic acid or in buffers at various pHs.
D. A 7% levodopa/2% carbidopa solution was prepared by adding pre-heated 0.1% Na-bisulfite solution to arginine. Levodopa was added to obtain a final molar ratio of 1:2 LD:Arg. The mixture was stirred at 75-80°C until complete dissolution was obtained. After cooling down to 60°C, carbidopa and arginine were added to obtain a final molar ratio of 1:1.2 CD(carbidopa):Arg(arginine). The mixture was stirred at 60°C until complete dissolution was obtained. After cooling, about 12.5% more arginine was added to the solution. The pH of the solution was about 9.2.
E. A 7% weight percent levodopa solution was prepared by adding pre-heated 0.1% Na-bisulfite solution to arginine. Levodopa was added to obtain a final molar ratio of 1:2 LD:Arg. The mixture was stirred at 75-80°C until complete dissolution was obtained. After cooling down, the pH of the solution was about 9.4.
F. A 2% or 4% entacapone or tolcapone solution was prepared by dissolving entacapone or tolcapone in a solution containing 1 equivalent of meglumine (molar ratio of entacapone/tolcapone:meglumine was 1:1) at a pH of 8.23.

### Example 2. Formulation preparation procedure

Levodopa (LD) and carbidopa (CD) formulations can be prepared as follows. However, as shown in Table A, the method of preparation has significant impact on the resulting physical and chemical stability of the composition.

***Method 1 (L-Arg solution).*** L-Arg and Na-Bis (Na-bisulfate) were dissolved in water. The solution was added to the LD and CD powders. The mixture was heated with stirring for 13 min at 75°C until fully dissolved. LD/CD solution was kept at RT for 10 min to cool down.

***Method 2 (all powders together).*** All powders (LD, CD and L-Arg) were weighed and water with Na-Bis was added. Suspension was heated with stirring for 13 min at 75°C until fully dissolved. LD/CD solution was kept at RT for 10 min to cool down.

***Method 3 (same as 2 without Na-Bis pre-heating).*** All powders (LD, CD and L-Arg) were weighed together and water was added. Suspension was heated with stirring for 13 min at 75°C until fully dissolved. LD/CD solution was kept at RT for 10 min to cool down.

***Method 4 (preparation in steps).*** LD and the respective amount of L-Arg were weighed; water and Na-Bis solution were added. The suspension was heated for 7 min at 75°C until fully dissolved followed by 7 min at RT. CD and the respective amount of L-Arg were weighed, and added to the LD/Arg solution at 60°C until fully dissolved. Finally, extra L-Arg was added.

***Method 5 (same as 4 without Na-Bis pre-heating).*** LD and the respective amount of L-Arg were weighed; water was added. The suspension was heated for 7 min at 75°C until fully dissolved followed by 7 min at RT. CD and the respective amount of L-Arg were weighed, and added to the LD/Arg solution at 60°C until fully dissolved. Finally, extra L-Arg was added.

After cooling down, all formulations from all methods were divided in to 3 vials, and water, Na-Bis solution or Na-Bis-Arg solution was added to each vial. The physical and chemical stability were evaluated and are presented in Tables A1 and A2.

**Table A1 - Physical stability**

| Method | | First test | | | Second test | |
|---|---|---|---|---|---|---|
| | | 24 hours | 48 hours | 72 hours | 24 hours | 48 hours |
| 1 | Water | +++ | NR | NR | ++ | NR |
| | Na-Bis solution | +++ | | | ++ | |
| | Na-Bis solution titrated with L-Arg | +++ | | | ++ | |
| 2 | Water | + | ++ | NR | - | +/- |
| | Na-Bis solution | - | + | | - | +/- |
| | Na-Bis solution titrated with L-Arg | - | +/- | | - | +/- |
| 3 | Water | - | - | + | - | Very few particles at the bottom |
| | Na-Bis solution | - | - | + (more than 13,15) | - | |
| | Na-Bis solution titrated with L-Arg | | - | + | | |
| 4 | Water | + | NR | NR | + | NR |
| | Na-Bis solution | + | | | + | |
| | Na-Bis solution titrated with L-Arg | +/- | | | + | |
| 5 | Water | ++ | NR | NR | + | NR |
| | Na-Bis solution | ++ | | | + | |
| | Na-Bis solution titrated with L-Arg | ++ | | | + | |

| | | | | | | |
|---|---|---|---|---|---|---|
| - No precipitate + Precipitate | | | | | | |

The formulations were sampled for HPLC analysis at the end of the preparation and after 5 days at RT. The recovery after 5 days at RT was calculated and compared to T=0.

The results in Table A1 and A2 clearly show that the method of formulation preparation has a significant impact on its physical and chemical stability. The formulation of Method 3 shows significantly more stability.

**Table A2 - Chemical stability**

| Method | | First test | | Second test | |
|---|---|---|---|---|---|
| | | LD recovery after 5 days (%) | CD recovery after 5 days (%) | LD recovery after 5 days (%) | CD recovery after 5 days (%) |
| 1 | Water | 90.6 | 98.0 | 89.5 | 100.4 |
| | Na-Bis solution | 90.6 | 98.6 | 87.0 | 101.3 |
| | Na-Bis solution titrated with L-Arg | 90.8 | 98.0 | 88.9 | 99.9 |
| 2 | Water | 98.4 | 98.2 | 99.1 | 100.1 |
| | Na-Bis solution | 98.2 | 98.1 | 99.4 | 100.5 |
| | Na-Bis solution titrated with L-Arg | 99.0 | 98.5 | 98.9 | 99.5 |
| 3 | Water | 99.7 | 97.5 | 95.5^{[a]} | 96.5 |
| | Na-Bis solution | 99.2 | 97.7 | 97.7^{[b]} | 99.1 |
| | Na-Bis solution titrated with L-Arg | 99.5 | 98.1 | 94.9^{[b]} | 96.2 |
| 4 | Water | 97.7 | 97.5 | 96.3 | 99.3 |
| | Na-Bis solution | 96.0 | 95.8 | 94.9 | 97.6 |
| | Na-Bis solution titrated with L-Arg | 97.7 | 97.9 | 96.3 | 100.0 |
| 5 | Water | 97.9 | 96.3 | 98.1 | 100.9 |
| | Na-Bis solution | 98.2 | 98.0 | 98.2 | 102.2 |
| | Na-Bis solution titrated with L-Arg | 97.4 | 96.7 | 98.3 | 100.6 |

| | | | | | |
|---|---|---|---|---|---|
| ^{[a]} The recovery values were lower at the second test compared to the first test, due to technical problem which occurred during the sampling. ^{[b]} The recovery values were lower at the second test compared to the first test, due to technical problem which occurred during the sampling. | | | | | |

### Example 3. Effect of arginine on long term stability of levodopa and levodopa/carbidopa compositions

Liquid formulations with levodopa, carbidopa and arginine were prepared using the procedure outlined in Example 2, and comparative studies on formulations with a different concentration of arginine and/or an amino sugar (e.g., meglumine), and /or a sugar (e.g. dextrose), and/or a base (NaOH), or another basic amino acid (e.g., lysine, histidine) were prepared. The results are shown in Table B.

**Table B**

| LD/CD Conc. (%) | Amino Acid (AA) | | | Other | | | Dissolution | Physical stability at RT |
|---|---|---|---|---|---|---|---|---|
| | Name | Conc. (%) | Molar ratio (API:Arg) | Name | Conc. (%) | Molar ratio (API:CI) | | |
| 10/0 | Lys | 8.5 | 1:2.5 | - | - | - | No | NA |
| 5/0 | Lys | 9.25 | 1:2.5 | - | - | - | No | NA |
| 3.3/0 | Lys | 6.2 | 1:2.5 | - | - | - | No | NA |
| 3/0 | Lys | 5.6 | 1:2.5 | - | - | - | Partial | NA |
| 2.5/0 | Lys | 4.6 | 1:2.5 | - | - | - | Yes | 2 days |
| 5/0 | His | 9.8 | 1:2.5 | - | - | - | No | NA |
| 2.5/0 | His | 4.9 | 1:2.5 | - | - | - | No | NA |
| 1.25/0 | His | 2.5 | 1:2.5 | - | - | - | Yes | 14 days |
| 9/0 | Arg | 8.2 | 1:1 | - | - | - | No | NA |
| 4.7/0 | Arg | 4.0 | 1:1 | - | - | - | No | NA |
| 9.5/0 | Arg | 15.9 | 1:1.9 | - | - | - | Yes | 2 days |
| 4.8/1.4 | Arg | 11.0 | 1:2.0 | - | - | - | Yes | ≥2 months |
| 4.8/1.4 | Arg | 12.1 | 1:2.2 | - | - | - | Yes | ≥2 months |
| 4.8/1.4 | Arg | 12.7 | 1:2.4 | - | - | - | Yes | ≥2 months |
| 5.4/1.5 | Arg | 13.5 | 1:2.1 | - | - | - | Yes | ≥2 months |
| 5.4/1.5 | Arg | 14.8 | 1:2.3 | - | - | - | Yes | ≥2 months |
| 6/1.5 | Arg | 14.8 | 1:2.1 | - | - | - | Yes | ≥1 month |
| 6/1.5 | Arg | 16.0 | 1:2.3 | - | - | - | Yes | ≥2 months |
| 7/2 | Arg | 17.8 | 1:2.2 | - | - | - | Yes | ≥1 month |
| 7/1.5 | Arg | 14.1 | 1:1.8 | Dex | 5.0 | - | Yes | Color change |
| 8/1.5 | Arg | 15.7 | 1:1.9 | Dex | 5.0 | - | Yes | Color change |
| 10/1.5 | Arg | 19.2 | 1:1.9 | Dex | 5.0 | - | Yes | Color change |
| 6/1.5 | Arg | 9.3 | 1:1.5 | NaOH | 4.6 | 1:0.5 | Yes | ≥3 months |
| 5/0 | - | - | - | Meg | 5.0 | 1:1 | No | NA |
| 5/0 | - | - | - | Meg | 5.9 | 1:1.2 | No | NA |
| 5/0 | - | - | - | Meg | 10.8 | 1:2.2 | Yes | NA |
| 8/1.5 | Arg | 15.7 | 1:1.9 | Meg | 3.2 | 1:0.4 | Yes | ≥4.5 months |
| 8/1.5 | Arg | 12.2 | 1:1.5 | Meg | 7.9 | 1:1 | Yes | ≥4.5 months |
| 10/1.5 | Arg | 19.2 | 1:1.9 | Meg | 4.0 | 1:0.4 | Yes | ≥4.5 months |
| 10/1.5 | Arg | 14.6 | 1:1.5 | Meg | 9.9 | 1:1 | Yes | ≥4.5 months |
| 7/1.5 | Arg | 14.1 | 1:1.9 | Meg | 2.8 | 1:0.4 | Yes | ≥4.5 months |
| 7/1.5 | Arg | 10.7 | 1:1.5 | Meg | 6.9 | 1:1 | Yes | ≥4.5 months |

| | | | | | | | | |
|---|---|---|---|---|---|---|---|---|
| * Lys - lysine; His - histidine; Arg - arginine; Dex - dextrose; Meg - meglumine; NA - not available. | | | | | | | | |

Table B indicates that arginine forms stable solutions with high concentrations of levodopa and carbidopa (>2.5%) at molar ratios <1:2.5, whereas with other basic amino acids LD does not even dissolve under these conditions. At molar ratios of LD/CD to arginine 1:<2, the solutions do not have long term stability, unless meglumine or another counterion is used, and meglumine may be used to reduce the molar ratio of arginine to LD/CD.

Liquid formulations were prepared by weighing all powders (LD, CD and L-Arg) and the addition of water pre-heated to 73±3°C. Suspension was put in a water bath at 73±3°C and stirred for 10 min until fully dissolved. LD/CD solution was kept at RT for 10 min to cool down. Then, ascorbic acid was added. Solutions were divided into glass vials and kept at +25°C and at -20°C for the indicated period of time. Prior to analyses, frozen vials were placed at RT until fully thawed. Formulations were then mixed and subjected to stability analyses.

Tables C1-C6 indicate the effect of L-Arg on physical and chemical long term stability at +25°C and at -20°C. In particular, these Tables show that there is a correlation between the molar ratio of arginine to LD/CD and stability where generally compositions having more arginine, have longer stability: LD/CD:arginine solutions (at molar ratios of 1:≥2.1) are stable for at least 1 month at RT and at -20±5°C. The solutions are stable even at very high solid concentrations (total of >45%).

**Table C1**

| Formulation | L-Arg concentration (%) | Physical stability at RT | Stability (% from T=0) at RT | | | |
|---|---|---|---|---|---|---|
| | | | 5 | | 2 months | |
| | | | LD | CD | LD | CD |
| 6/1.5% LD/CD (1% Na-Asc) | 13.5 | 6 days | 100.0 | 97.5 | | |
| | 14.2 | At least 7 days | 100.8 | 96.7 | | |
| | 14.8 | | 99.6 | 96.6 | | |
| | 16.0 | | 99.5 | 96.6 | | |
| 4.8/1.4% LD/CD (1% Na-Asc) | 11.0 | At least 2 months | 99.4 | 97.3 | 100.1 | 93.7 |
| | 11.6 | | 98.9 | 97.4 | 100.6 | 96.2 |
| | 12.1 | | 99.1 | 97.0 | 100.3 | 94.3 |
| | 12.7 | | 99.4 | 97.2 | 99.0 | 92.4 |

**Table C2**

| Formulation | L-Arg concentration (%) | Physical stability | Stability (% from T=0) 2 weeks at -20±5°C | | | |
|---|---|---|---|---|---|---|
| | | | Immediately after thawing | | 24 hours at RT | |
| | | | LD | CD | LD | CD |
| 6/1.5% LD/CD (1% Na-Asc) at -20°C | 13.5 | At least 24h after thawing | 99.7 | 98.4 | 100.0 | 99.1 |
| | 14.2 | | 99.8 | 98.1 | 101.0 | 99.4 |
| | 14.8 | | 100.0 | 98.9 | 99.9 | 98.9 |
| | 16.0 | | 99.9 | 98.8 | 100.3 | 99.3 |

**Table C3**

| Formulation | L-Arg concentration (%) | Physical stability (at RT) | |
|---|---|---|---|
| | | 1% Na-Asc | 1% Asc |
| 6/1.5% LD/CD | 14.8 | At least 3 weeks | At least 3 days |
| | 15.8 | | |
| | 16.8 | | |
| 5.4/1.5% LD/CD | 12.3 | At least 3 days | |
| | 13.5 | | |
| | 14.8 | | |

**Table C4**

| Formulation | L-Arg conc. (%) | Physical stability (after 2 month at RT) | Stability (% from T=0) at RT | | | | | |
|---|---|---|---|---|---|---|---|---|
| | | | 1 weeks | | 2 weeks | | 1 month | |
| | | | LD | CD | LD | CD | LD | CD |
| 5.4/1.5% LD/CD (1% Asc) | 13.5 | + | 101.4 | 100.4 | 101.7 | 98.4 | 98.8 | 103.1 |
| | 14.8 | + | 101.4 | 101.4 | 102.0 | 100.1 | 99.0 | 104.2 |
| 6/1.5% LD/CD (1% Asc) | 14.8 | + | 101.8 | 101.5 | 101.6 | 99.6 | 99.0 | 104.2 |
| | 16.0 | - | 101.1 | 100.4 | 102.8 | 100.6 | 99.4 | 104.2 |
| 7/2% LD/CD (1% Asc) | 17.8 | + | 101.7 | 101.0 | 102.7 | 99.7 | 98.7 | 103.1 |
| 7/2% LD/CD (1% Na-Asc) | | - | 100.6 | NA | 101.9 | 99.2 | 98.4 | 103.6 |

**Table C5**

| Formulation | L-Arg conc. (%) | Physical stability (11 days after thawing) | Stability (% from T=0) 2 weeks at -20±5°C, immediately after thawing | | Stability (% from T=0) 5 weeks at -20±5°C, immediately after thawing | |
|---|---|---|---|---|---|---|
| | | | LD | CD | LD | CD |
| 5.4/1.5% LD/CD (1% Asc) | 13.5 | + | 102.3 | 99.5 | 99.4 | 104.3 |
| | 14.8 | - | 102.7 | 101.3 | 99.6 | 104.6 |
| 6/1.5% LD/CD (1% Asc) | 14.8 | - | 102.6 | 101.1 | 99.1 | 104.2 |
| | 16.0 | - | 103.2 | 100.9 | 99.2 | 104.3 |
| 7/2% LD/CD (1% Asc) | 17.8 | + | 102.8 | 101.0 | 99.2 | 104.3 |
| 7/2% LD/CD (1% Na-Asc) | | - | 102.9 | 101.0 | 99.4 | 104.4 |

**Table C6**

| LD/CD concentration | L-Arg concentration (%) | Physical stability at 25°C |
|---|---|---|
| 12/3% | 24.4 | Considerable precipitate on Day 5 |
| | 29.6 | Slight precipitate on Day 5 |
| | 32.1 | No precipitate on Day 7 |

Formulations containing 6/1.5% and 5.4/1.5% LD/CD and varying L-Arg concentrations were titrated with acetic acid (100%) or lactic acid (85%), to study the effect of pH and L-Arg concentration on the physical stability of the solutions (Table D).

**Table D**

| | L-Arginine (%) | Asc/Na-Asc | pH before | Lactic (%) | pH after lactic | pH drop | 4 hours | 24 hours | | |
|---|---|---|---|---|---|---|---|---|---|---|
| | 14.8 | Na-Asc | 9.53 | 1.1 | 9.25 | -0.28 | OK | + | | |
| | | | 9.53 | 1.7 | 9.16 | -0.37 | + | + | | |
| | | | 9.53 | 2.3 | 9.02 | -0.51 | ++ | + | | |
| | 14.8 | Asc | 9.41 | 0.85 | 9.24 | -0.17 | OK | + | | |
| | | | 9.42 | 1.3 | 9.14 | -0.28 | + | + | | |
| | | | 9.41 | 1.7 | 9.06 | -0.35 | + | + | | |
| | 15.8 | Na-Asc | 9.52 | 1.1 | 9.33 | -0.19 | OK | OK | | |
| 6/1.5% LD/CD | | | 9.50 | 1.7 | 9.21 | -0.32 | OK | + | | |
| | | | 9.53 | 2.3 | 9.08 | -0.45 | + | + | | |
| | 15.8 | Asc | 9.44 | 0.85 | 9.27 | -0.17 | OK | OK | | |
| | | | 9.45 | 1.3 | 9.19 | -0.26 | OK | + | | |
| | | | 9.45 | 1.7 | 9.11 | -0.34 | + | + | | |
| | 16.8 | Na-Asc | 9.56 | 1.1 | 9.36 | -0.20 | OK | OK | | |
| | | | 9.56 | 1.7 | 9.23 | -0.33 | OK | OK | | |
| | | | 9.56 | 2.3 | 9.09 | -0.47 | OK | + | | |
| | 16.8 | Asc | 9.46 | 0.85 | 9.30 | -0.16 | OK | OK | | |
| | | | 9.46 | 1.3 | 9.20 | -0.26 | OK | OK | | |
| | | | 9.47 | 1.7 | 9.11 | -0.36 | OK | + | | |

| | L-Arginine (%) | Asc/Na-Asc | pH before | Lactic (%) | Acetic (%) | pH after | pH drop | 2 days | 3 days | 10 days |
|---|---|---|---|---|---|---|---|---|---|---|
| | 12.3 | Na-Asc | 9.41 | 0.36 | - | 9.35 | -0.06 | OK | + | + |
| | | | 9.43 | 1.0 | - | 9.18 | -0.25 | ++ | + | + |
| | | | 9.43 | - | 0.35 | 9.29 | -0.14 | OK | + | + |
| | 12.3 | Asc | 9.28 | 0.36 | - | 9.20 | -0.08 | ++ | + | + |
| | | | 9.29 | 1.0 | - | 9.05 | -0.24 | ++ | ++ | ++ |
| | | | 9.29 | - | 0.35 | 9.14 | -0.15 | ++ | ++ | ++ |
| | 13.5 | Na-Asc | 9.50 | 0.36 | - | 9.38 | -0.12 | OK | OK | OK |
| 5.4/1.5% LD/CD | | | 9.48 | 1.0 | - | 9.25 | -0.23 | + | + | + |
| | | | 9.49 | - | 0.35 | 9.35 | -0.14 | OK | OK | OK |
| | 13.5 | Asc | 9.32 | 0.36 | - | 9.25 | -0.07 | + | + | + |
| | | | 9.33 | 1.0 | - | 9.11 | -0.22 | ++ | ++ | ++ |
| | | | 9.34 | - | 0.35 | 9.20 | -0.14 | + | + | + |
| | 14.8 | Na-Asc | 9.51 | 0.36 | - | 9.43 | -0.08 | OK | OK | OK |
| | | | 9.51 | 1.0 | - | 9.28 | -0.23 | OK | OK | OK |
| | | | 9.51 | - | 0.35 | 9.38 | -0.13 | OK | OK | OK |
| | 14.8 | Asc | 9.36 | 0.36 | - | 9.29 | -0.07 | OK | OK | OK |
| | | | 9.37 | 1.0 | - | 9.13 | -0.24 | +/- | + | + |
| | | | 9.36 | - | 0.35 | 9.23 | -0.13 | OK | OK | OK |

| | | | | | | | | | | |
|---|---|---|---|---|---|---|---|---|---|---|
| * OK - no precipitate; +/- very few particles; + slight precipitate; ++ considerable precipitate | | | | | | | | | | |

Table D indicates that ascorbic acid reduces the pH by 0.1-0.15 units as compared to Na-ascorbate and that other organic acids can further reduce the pH of the formulations. But the physical stability test results indicate that formulations are not generally stable at pH <9.15±0.5. Formulations with Na-ascorbate appear more stable than formulations with ascorbic acid at a given L-arginine concentration. Thus, it is suggested that excess of acid may cause precipitation in the absence of adequate amount of L-Arg.

Table E shows the physical and chemical stability 3 weeks post-preparation of the 6/1.5/14.8% LD/CD/Arg formulation used for the stability tests shown in Table D.

**Table E**

| Formulation | Asc/Na-Asc (1%) | Physical stability (at RT) | Stability (% of T₀) | |
|---|---|---|---|---|
| | | | LD | CD |
| 6/1.5% LD/CD, 14.8% L-Arg | Asc | ≥3 weeks | 103.1 | 98.9 |
| | Na-Asc | | 101.1 | 97.4 |

### Example 4: Stability of levodopa formulations with carbidopa in-vitro and ex-vivo

The effect of carbidopa on levodopa formulations was investigated. Levodopa (LD) formulations were prepared with 0, 0.5, 1, 1.5 and 2% by weight carbidopa (CD) and a constant concentration of arginine. Physical and chemical stabilities were evaluated, as shown in Table F.

**Table F**

| Formulation | | N2 +/- | Physical stability | Stability (% from T=0) | | | |
|---|---|---|---|---|---|---|---|
| | | | | 3 days | | 15 days | |
| | | | | LD | CD | LD | CD |
| 7% LD | w/o CD | + | Stable | 99.2 | NA | 103.4 | NA |
| | | - | Stable | 98.1 | NA | - | NA |
| | 0.5% CD | + | Stable | 98.6 | 94.7 | 104.1 | 108.1 |
| | | - | Stable | 98.7 | 95.6 | - | - |
| | 1% CD | + | Stable | 98.9 | 95.2 | 102.5 | 104.4 |
| | | - | Slight precipitate | 97.9 | 94.0 | - | - |
| | 1.5% CD | + | 7 days | 98.1 | 94.2 | 103.7 | 104.8 |
| | | - | | 99.6 | 96.0 | - | - |
| | 2% CD | + | 4 days | 98.9 | 94.5 | 102.9 | 103.3 |
| | | - | | 98.3 | 94.8 | - | - |

The experimental results shown in **Fig. 1A** indicate that carbidopa prevented dark yellow color formation in the presence of air, in a dose related manner. In the absence of air (with N₂ in the head space) 0.5% CD was sufficient to inhibit this color formation. It is suggested that CD inhibits oxidation of LD *in vitro.* The experimental results shown in Table F indicate that carbidopa does not have a significant effect on the chemical stability of levodopa. It also shows that the ratio between arginine and the total active ingredients is important to prevent precipitation, i.e., the physical stability of the formulation is depended on the relative concentration of arginine

In an additional experiment, LD formulations were prepared with 0, 0.5, 1 and 2% CD and respective concentrations of arginine. Physical and chemical stability were evaluated, and results are shown in Table G.

**Table G**

| Formulation | L-Arg (%) | Chemical stability at RT (% of t₀) | | | | Physical stability at RT 1 month after thawing LD |
|---|---|---|---|---|---|---|
| | | 3 days | | 1 month after thawing | | |
| | | LD | CD | LD | CD | |
| 6% LD / 0% CD | 13.5 | 102.3 | - | 6%LD / 0%CD | 13.5 | 102.3 |
| 6% LD / 0.5% CD | 14.2 | 103.3 | 100.4 | 6%LD / 0.5%CD | 14.2 | 103.3 |
| 6% LD/ 1% CD | 14.8 | 103.5 | 101.3 | 6%LD / 1%CD | 14.8 | 103.5 |
| 6% LD / 2% CD | 16.5 | 103.3 | 101.6 | 6%LD / 2%CD | 16.5 | 103.3 |

In the presence of adequate concentrations of L-arginine, all formulations *ex-vivo* were stable for at least a month at RT following thawing, as shown in Table G.

The effect of carbidopa on the stability of levodopa formulations is shown in **Fig. 1****.** A 7% LD/arginine solution, with or without 2% CD, was continuously administered at 0.08 ml/h ×18h, 37° C into a 5×5cm fresh, full-thickness pig skin. The right hand side of **Fig. 1** indicates the lack of black by-products formation, suggesting that CD inhibits oxidation of LD *ex vivo* and may also inhibit the formation of o-quinones and melanin.

### Example 5. Stability of carbidopa formulations with levodopa

The effect of levodopa on the stability of carbidopa was investigated. Table H indicates results.

Table H indicates that CD was less sensitive to oxidation and degradation and was more stable in the presence of LD: the area of impurities at retention time (R.T.) 4.82, 5.65, 12.7, 13.53 and 14.55 were significantly increased under aerobic conditions when LD was not present, and the area of impurities at R.T. at 4.82 and 13.53 were increased even in the absence of oxygen. It appears that LD may protect CD from degradation.

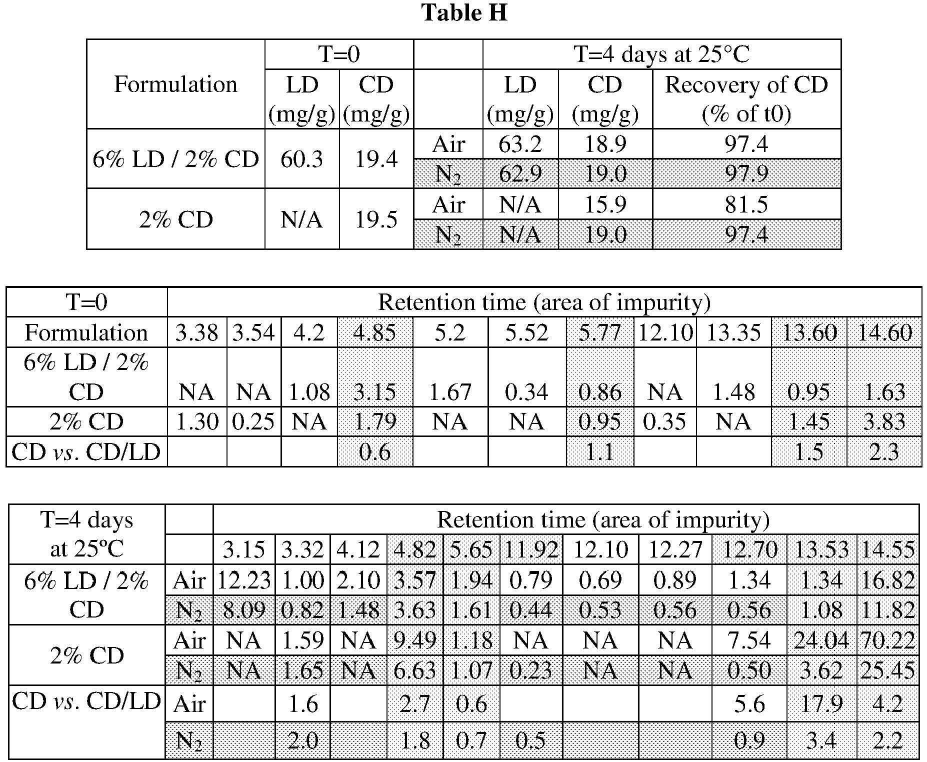

### Example 6. Toxicity and pharmacokinetics of levodopa formulations with carbidopa

The effect of carbidopa on levodopa local toxicity was investigated in pigs: solutions containing 6% LD and 0, 0.5 or 1% CD with the respective amount of arginine (13.5, 14.2 or 14.8%, respectively) were continuously administered SC to pigs at 0.16 ml/h ×24h. Each formulation was administered to 2 pigs. Skin samples were collected 8±1 days thereafter. **Fig. 2** shows that the presence of 1% carbidopa reduces the severity and extent of levodopa dependent toxicity, *in-vivo.*

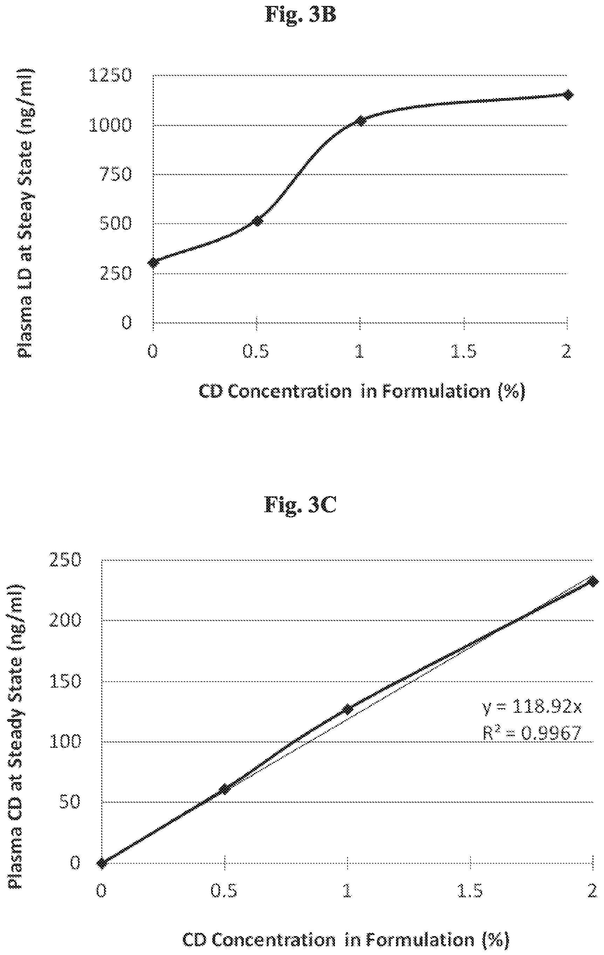

The effect of carbidopa on the pharmacokinetics of levodopa and carbidopa were investigated. Solutions containing 6% LD and 0, 0.5, 1 or 2% CD and the respective amount of arginine (13.5, 14.2, 14.8 or 16.5%, respectively) were continuously administered SC to pigs at 0.16 ml/h ×24h. **Fig. 3** shows that CD has a significant effect on the pharmacokinetics of LD. This effect was dose dependent and linear between ±0.3 and ±1.2% CD.

### Example 7. Plasma levels of levodopa following subcutaneous administration

In this experiment, the purpose was to determine the plasma levels of LD (levodopa) following continuous subcutaneous administration of carbidopa, levodopa or entacapone and combinations thereof with oral LD/CD in pigs.

Landrace × Large White female pigs weighing about 22 kg were treated, starting on Day 1 at 15:00 as per Table I, with oral LD/CD 100/25 and with the respective test formulations, containing carbidopa, levodopa or entacapone and combinations thereof, formulated with arginine, as described above, and administered continuously subcutaneously via a dermal patch (Omnipod®) at a rate of 0.08 ml/h.

Table I indicates the treatment protocol of each group. The formulations were prepared as in Example 1 and 2.

**Table I**

| Treatment group | None | CD | CD+E | E | LD+CD | LD |
|---|---|---|---|---|---|---|
| *n* | *3* | *3* | *3* | *2* | *2* | *1* |
| SC route of administration | No SC treatment | 2% carbidopa | 2% carbidopa + 10% entacapone | 10% entacapone | 7% levodopa + 2% carbidopa | 7% levodopa |
| Oral treatment | 100/25 levodopa/carbidopa | | | | | |

Blood samples were collected following the 3^{rd} oral dose at pre-determined time points and plasma levels of levodopa, carbidopa and 3-OMD were analyzed by HPLC-ECD.

**Fig. 4** indicates the mean levodopa plasma concentrations following oral administration of Sinemet (oral 100/25 LD/CD) with continuous SC administration of entacapone (200 mg/24h) ±CD (40mg/24h), as two separate compositions **(****Fig. 4A****);** or LD (140 mg/24h) ±CD (40mg/24h) in pigs **(****Fig. 4B****)** (all subcutaneous formulations included arginine, as above).

Results show that there is a synergistic effect between entacapone (200 mg/24h) and CD (40mg/24h) on the plasma pharmacokinetics (PK) of levodopa (ng/ml) when co-administered continuously subcutaneously, as compared to the calculated LD plasma PK obtained after adding the plasma concentrations of LD following the continuous SC administration of CD and entacapone each alone (**Fig. 1A** and Table 2, C *vs.* B+D). Results also show that there is an additive effect between levodopa (140 mg/24h) and CD (40mg/24h) on the plasma PK of levodopa (ng/ml) when co-administered continuously subcutaneously, as compared to the calculated LD plasma PK obtained after adding the plasma concentrations of LD following the continuous SC administration of CD and LD each alone (**Fig. 1B** and Table 2, E *vs.* D+F). Moreover, the results suggest that continuous SC administration of LD and CD may be sufficient to maintain constant, continuous levodopa plasma concentrations even in the absence of oral LD/CD administration (**Fig. 4B** dotted line and Table J 'E minus A'). Table J presents concentrations of plasma levodopa 6½ and 8h post-oral LD/CD administration.

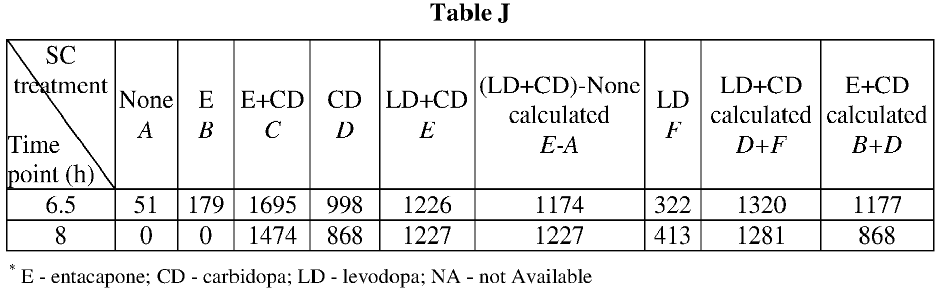

**Fig. 5** shows tissue biopsies from the application site of the levodopa-carbidopa arginine combination formulation and the levodopa/arginine formulation. No visible tissue irritation or damage was apparent in the levodopa-carbidopa arginine formulation. The site administered with levodopa-arginine formulation appears to have some blackening of tissue. Without being limited by any theory, it is thought that having carbidopa and arginine together with levodopa (arginine) formulation protects the local tissue from local damage of levodopa by preventing oxidation of levodopa into irritant by products, and that carbidopa is a potent anti-oxidant.

### Example 8. Additional exemplary carbidopa and levodopa/carbidopa formulations

In Tables K and L, additional non-limiting exemplary carbidopa and levodopa/carbidopa formulations are provided.

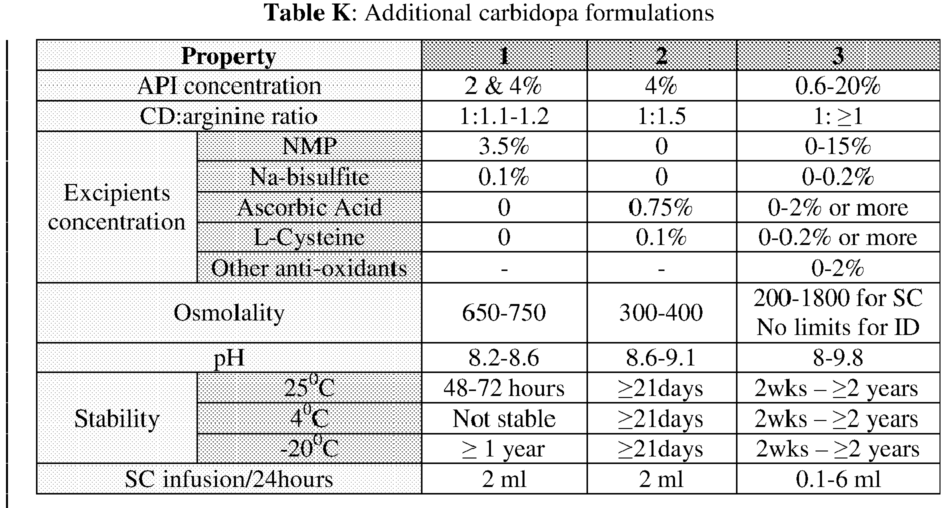

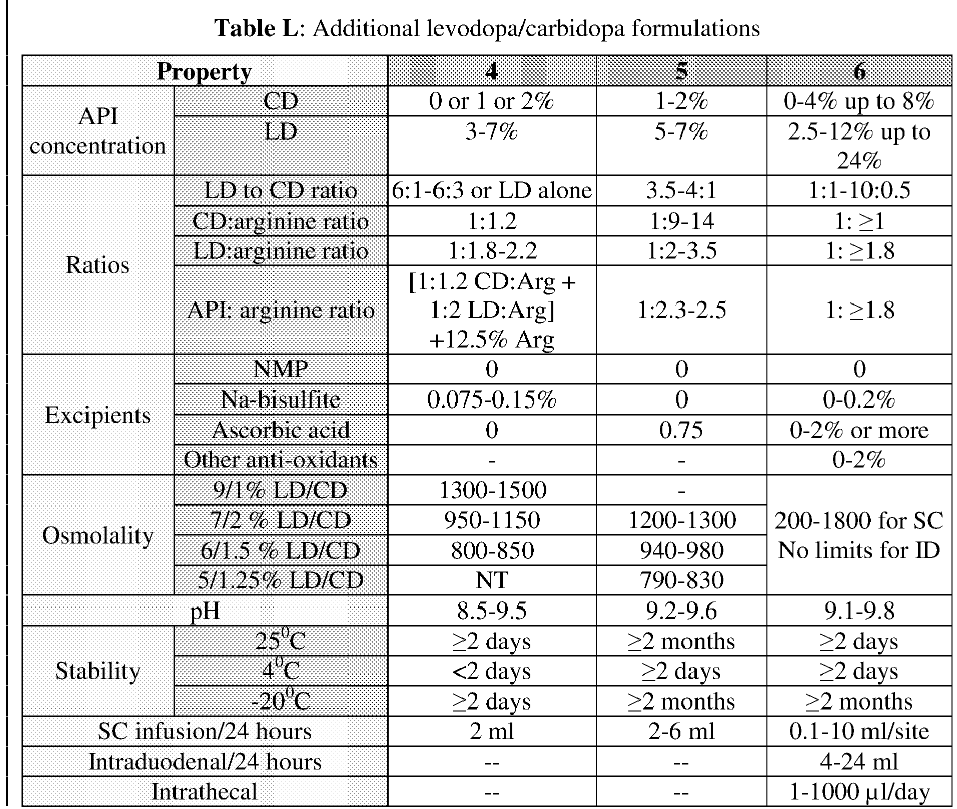

### Example 9. Plasma levels of levodopa, carbidopa, and 3-O-methyldopa following continuous subcutaneous administration of carbidopa and levodopa and oral administration of entacapone

In this experiment, the purpose was to determine the plasma levels of levodopa (LD), carbidopa (CD), and 3-O-methyldopa (3-OMD) following continuous subcutaneous administration of carbidopa and levodopa, and oral administration of entacapone in human volunteers.

A single center, double-blind, randomized, placebo-controlled, study was conducted with six healthy Caucasian males volunteers, aged 18-40 years old. LD (6%) /CD (1.5%) was administered at a rate of 240 µl/h, corresponding to 360 mg of LD and 90 mg of CD per 24 hours. Entacapone (200 mg) was administered orally every 2 hours, starting 15h after infusion initiation of LD/CD. Plasma LD, CD and 3-OMD concentrations were quantified at pre-determined time points.

As shown in **Fig. 6****,** the results demonstrate that oral entacapone increased the plasma concentrations of LD attainable with the continuous SC administration of LD/CD by 50% within 9 hours after the initiation of entacapone dosing. The plasma concentrations of LD did not reach a steady state by the time the study was terminated 24 hours after LD/CD infusion initiation and 9 hours after entacapone dosing initiation. The plasma concentrations of 3-OMD were significantly reduced. These results suggest that rather than administering 200 mg of entacapone 6-8 times daily, as is typically done with oral LD/CD administration, 400 mg entacapone can instead be administered, e.g., twice or three times daily.

### EQUIVALENTS

While specific embodiments of the subject invention have been discussed, the above specification is illustrative and not restrictive. Many variations of the invention will become apparent to those skilled in the art upon review of this specification. The full scope of the invention should be determined by reference to the claims, along with their full scope of equivalents, and the specification, along with such variations.

Unless otherwise indicated, all numbers expressing quantities of ingredients, reaction conditions, and so forth used in the specification and claims are to be understood as being modified in all instances by the term "about". It should be noted that where particular values are described in the description and claims, unless otherwise stated, the term "about" means that an acceptable error range, e.g., up to 5% or 10%, for the particular value should be assumed.

### INCORPORATION BY REFERENCE

The entire contents of all patents, published patent applications, websites, and other references cited herein are hereby expressly incorporated herein in their entireties by reference.

The present invention includes the following embodiments:
1. A method for treatment of Parkinson's disease in an individual in need thereof comprising parenterally administering a pharmaceutical composition comprising carbidopa and levopoda to said individual; and orally administering a catechol-O-methyl transferase (COMT) inhibitor.
2. The method of item 1, wherein said composition is administered subcutaneously, transdermally, intradermally, intravenously, intramuscularly, intratracheally, intranasally, intrathecally, intragastrically or intraduodenally.
3. The method of item 1, wherein said composition is administered in one or more sites on the individual's body.
4. The method of any one of items 1 to 3, wherein said composition is substantially continuously administered.
5. The method of item 4, wherein said composition further comprises arginine.
6. The method of item 5, wherein said composition has a molar ratio of carbidopa and levodopa to arginine of about 1:2 to about 1:3.5.
7. The method of item 6, wherein said composition comprises (i) arginine, about 0.1% to about 2% by weight carbidopa, and about 4% to about 8% by weight levodopa; or (ii) arginine, about 0.6% to about 1.5% by weight carbidopa, and about 6% by weight levodopa.
8. The method of item 7, wherein said composition is administered at a rate of about 0.1 to about 1000 µl/h/site; or at a volume of about 2 to about 10, preferably about 4 to about 6, ml/24h/site; or at a dose of about 80 to about 800 mg levodopa/day and about 20 to about 200 mg carbidopa/day; or at a rate of about 240 to about 360 mg levodopa and about 60 to about 90 mg carbidopa/day/site.
9. The method of item 8, wherein said composition is administered subcutaneously, transdermally, intradermally, intravenously, intramuscularly, intratracheally, intranasally or intrathecally.
10. The method of item 6, wherein said composition comprises (i) arginine, about 1% to about 4% by weight carbidopa, and about 6% to about 16% by weight levodopa; or (ii) arginine, about 1.5% to about 2.5% by weight carbidopa, and about 12% by weight levodopa.
11. The method of item 10, wherein said composition is administered at a rate of about 0.2 to about 2000 µl/h/site; or at a volume of about 10 to about 24, preferably about 12 to about 16 ml/24h/site; or at a dose of about 600 to about 4000 mg levodopa/day and about 60 to about 500 mg carbidopa/day; or at a rate of about 800 to about 1600 mg levodopa and about 200 to about 400 mg carbidopa/day/site.
12. The method of item 11, wherein said composition is administered intragastrically or intraduodenally.
13. The method of any one of items 1 to 12, wherein said COMT inhibitor is entacapone or tolcapone, and is administered 1, 2, 3, 4 or 5 times a day.
14. The method of item 13, wherein said COMT inhibitor is entacapone, and is administered at a dose of about 200 mg to about 600 mg, preferably about 400 mg, twice or three times a day.
15. The method of item 14, wherein the entacapone is administered twice a day.
16. The method of item 13, wherein said COMT inhibitor is tolcapone, and is administered at a dose of about 50 mg to about 200 mg, preferably about 100 mg, once or twice or three times a day.
17. The method of item 16, wherein the tolcapone is administered twice a day.
18. Carbidopa, levodopa and a COMT inhibitor for use as a combination in treatment of Parkinson's disease, wherein the carbidopa and the levodopa are formulated as a sole parenteral composition, and said COMT inhibitor is formulated as an oral composition.
19. The use of item 18, wherein the carbidopa and the levodopa are formulated as a subcutaneous, transdermal, intradermal, intravenous, intramuscularly, intratracheally, intranasally, intrathecal, intragastrical or intraduodenal composition.
20. The use of item 18, wherein said parenteral composition is suitable for substantially continuous administration.
21. The use of any one of items 18 to 20, wherein said parenteral composition further comprises arginine.
22. The use of item 21, wherein the composition has a molar ratio of carbidopa and levodopa to arginine of about 1:2 to about 1:3.5.
23. The use of item 22, wherein said composition comprises (i) arginine, about 0.1% to about 2% by weight carbidopa, and about 4% to about 8% by weight levodopa; (ii) arginine, about 0.6% to about 1.5% by weight carbidopa, and about 6% by weight levodopa; (iii) arginine, about 1% to about 4% by weight carbidopa, and about 6% to about 16% by weight levodopa; or (iv) arginine, about 1.5% to about 2.5% by weight carbidopa, and about 12% by weight levodopa.
24. The use of any one of items 18 to 23, wherein the COMT inhibitor is entacapone or tolcapone.

## Claims

1. Carbidopa, levodopa and a catechol-O-methyl transferase (COMT) inhibitor for use as a combination in treatment of Parkinson's disease, wherein the carbidopa and the levodopa are formulated as a sole parenteral composition, and said COMT inhibitor is formulated as an oral composition.

2. The carbidopa, levodopa and COMT inhibitor for use according to claim 1, wherein said composition is administered subcutaneously, transdermally, intradermally, intravenously, intramuscularly, intratracheally, intranasally, intrathecally, intragastrically or intraduodenally.

3. The carbidopa, levodopa and COMT inhibitor for use according to claim 1, wherein said composition is administered in one or more
sites on the individual's body.

4. The carbidopa, levodopa and COMT inhibitor for use according to any one of claims 1 to 3, wherein said composition is substantially continuously administered.

5. The carbidopa, levodopa and COMT inhibitor for use according to claim 4, wherein said composition further comprises arginine.

6. The carbidopa, levodopa and COMT inhibitor for use according to claim 5, wherein said composition has a molar ratio of carbidopa
and levodopa to arginine of about 1:2 to about 1:3.5.

7. The carbidopa, levodopa and COMT inhibitor for use according to claim 6, wherein said composition comprises (i) arginine, about 0.1% to about 2% by weight carbidopa, and about 4% to about 8% by weight levodopa; or (ii) arginine, about 0.6% to about 1.5% by weight carbidopa, and about 6% by weight levodopa.

8. The carbidopa, levodopa and COMT inhibitor for use according to claim 7, wherein said composition is administered at a rate of about 0.1 to about 1000 µl/h/site; or at a volume of about 2 to about 10, preferably about 4 to about 6, ml/24h/site; or at a dose of about 80 to about 800 mg levodopa/day and about 20 to about 200 mg carbidopa/day; or at a rate of about 240 to about 360 mg levodopa and about 60 to about 90 mg carbidopa/day/site.

9. The carbidopa, levodopa and COMT inhibitor for use according to claim 8, wherein said composition is administered subcutaneously, transdermally, intradermally, intravenously, intramuscularly, intratracheally, intranasally or intrathecally.

10. The carbidopa, levodopa and COMT inhibitor for use according to claim 6, wherein said composition comprises (iii) arginine, about 1% to about 4% by weight carbidopa, and about 6% to about 16% by weight levodopa; or (iv) arginine, about 1.5% to about 2.5% by weight carbidopa, and about 12% by weight levodopa.

11. The carbidopa, levodopa and COMT inhibitor for use according to claim 10, wherein said composition is administered at a rate of about 0.2 to about 2000 µl/h/site; or at a volume of about 10 to about 24, preferably about 12 to about 16 ml/24h/site; or at a dose of about 600 to about 4000 mg levodopa/day and about 60 to about 500 mg carbidopa/day; or at a rate of about 800 to about 1600 mg levodopa and about 200 to about 400 mg carbidopa/day/site.

12. The carbidopa, levodopa and COMT inhibitor for use according to claim 11, wherein said composition is administered intragastrically or intraduodenally.

13. The carbidopa, levodopa and COMT inhibitor for use according to any one of claims 1 to 12, wherein said COMT inhibitor is entacapone or tolcapone, and is administered 1, 2, 3, 4 or 5 times a day.

14. The carbidopa, levodopa and COMT inhibitor for use according to claim 13, wherein said COMT inhibitor is entacapone, and is administered at a dose of about 200 mg to about 600 mg, preferably about 400 mg, twice or three times a day, wherein the entacapone is preferably administered twice a day.

15. The carbidopa, levodopa and COMT inhibitor for use according to claim 13, wherein said COMT inhibitor is tolcapone, and is administered at a dose of about 50 mg to about 200 mg, preferably about 100 mg, once or twice or three times a day, wherein the tolcapone is preferably administered twice a day.
